# EUROPEAN PATENT SPECIFICATION

(11) **EP 3 870 119 B1**
(45) Date of publication and mention of the grant of the patent: **02.07.2025**
(21) Application number: 19874908.7
(22) Date of filing: 24.10.2019
(51) Int. Cl.: A61F 9/00, A61B 10/02

(54) **DEVICE FOR OPHTHALMIC EXTRACTION AND INJECTION**
VORRICHTUNG ZUR OPHTHALMISCHEN EXTRAKTION UND INJEKTION
DISPOSITIF D'EXTRACTION ET D'INJECTION OPHTALMIQUES

(30) Priority: 26.10.2018 SG 10201809514S
(43) Date of publication of application: 01.09.2021
(73) Proprietor: Singapore Health Services Pte. Ltd., Singapore 168753 (SG)
(72) Inventor: ANG, Han Nian Marcus, Singapore National Eye Centre 11 Third Hospital Avenue, Singapore 168751 (SG); TEO, Yi Chong Kelvin, Singapore National Eye Centre 11 Third Hospital Avenue, Singapore 168751 (SG); TAN, Cheng Sim Anna, Singapore National Eye Centre 11 Third Hospital Avenue, Singapore 168751 (SG)
(74) Representative: Zimmermann, Tankred Klaus
(86) International application number: PCT/SG2019/050525
(87) International publication number: WO 2020/086002

(56) References cited:
- WO-A1-2017/184084
- WO-A1-2017/184084
- WO-A2-2007/021671
- WO-A2-2013/003620
- CN-A- 101 732 792
- CN-A- 101 732 792
- CN-A- 106 730 169
- US-A- 5 716 363
- US-A1- 2015 305 721

## Description

### FIELD OF INVENTION

The present invention relates broadly, but not exclusively, to devices for ophthalmic extraction and injection.

### BACKGROUND

Age-Related Macular Degeneration (AMD) and diabetic retinopathy (DR) are the leading causes of blindness in the world, with an estimated projection of more than 66 million people affected by the year 2023. Currently, the standard of care for the treatment of AMD and DR includes monthly injections of Vascular Endothelial Growth Factor (VEGF) agents. Statistics from various eye healthcare centres in Singapore show that there are about 17,000 such injections per year.

However, there are problems associated with such injections. One problem may include ocular and systemic safety of such repeated injections over long periods of time, which are secondary to intraocular pressure (IOP) spikes (e.g. increase of 30mmHg). Another problem may include the inaccurate delivery of therapeutics due to reflux of vitreous material. Furthermore, there may also be no means of extracting intravitreal biomarkers, as current methods of extracting intravitreal biomarkers are merely proxies, i.e. having anatomical change (e.g. OCT) and functional change (e.g. vision). There are also problems when using typical injections, such as leak and reflux leading to Bleb formation, and damage to ocular structures.

It has also been noted that existing injection devices are not capable of extracting a sample from the eye, such that multiple punctures may be required for sample collection and drug delivery. The existing devices are also complicated and require trained doctors to operate, thus adding the cost of use to the drug cost.

WO 2013/003620 A2 discloses an apparatus to treat a patient, which comprises a container to receive fluid of a device implanted in the eye. An injector is coupled to a double lumen needle such that the lumen needle injects therapeutic agent from a chamber of a first container into a device. A second container is coupled to a first lumen that extends to a chamber of a reservoir container and receives liquid from the device, such that liquid of the device is exchanged. A piston can be moved toward the device with a plunger, and a sliding component of a switching valve can be coupled to the plunger and the piston. When the piston has advanced to exchange an intended amount of liquid and an intended amount of the formulation of the therapeutic agent remains in first container, the sliding component of valve may cover and block the opening component of valve. With valve closed, an additional intended amount of therapeutic agent can be injected into the device, such that a bolus amount of therapeutic agent can be injected from device.

Accordingly, a need exists to provide a device and method that seeks to address some of the above problems.

### SUMMARY

According to the present invention, there is provided an extraction and injection ophthalmic device comprising: a housing comprising: an injection portion configured to receive an injection source; an extraction portion configured to receive an extraction source; a priming mechanism configured to simultaneously prime both the injection source and the extraction source; a needle selectively in fluid communication with the injection source or the extraction source; wherein on activation of the extraction source, the device is configured to extract a sample through the needle, and on activation of the injection source the device is configured to inject an agent through the needle.

The priming mechanism further comprises a lever communicatively coupled to a spring, such that a translation movement of the lever is configured to compress the spring to simultaneously prime both the injection source and the extraction source.

In an embodiment, the device may further comprise a manifold configured to selectively connect the needle with the injection source or the extraction source, wherein the manifold comprises a double spool manifold.

In an embodiment, the device may further comprise shearing means configured to shear excess vitreous humor from the sample to be extracted.

In an embodiment, the injection source and the extraction source may be removably attached to the housing.

In an embodiment, the injection source may include a syringe.

In an embodiment, the extraction source may include any one of: a syringe, a vacutainer or a pump.

In an embodiment, the device may further comprise an injection button configured to activate the injection source such that the injection portion is in fluid communication with the needle to inject the agent from the injection source to the needle.

In an embodiment, the device may further comprise an extraction button configured to activate the extraction source such that the extraction portion is in fluid communication with the needle to extract the sample from the needle to the extraction source.

In an embodiment, the extraction source may be fluidically separated from the injection source such that, in use, the agent does not contact the sample.

A method for ophthalmic extraction and injection comprises the steps of: attaching an injection source to an injection portion of an extraction and injection ophthalmic device; attaching an extraction source to an extraction portion of the extraction and injection ophthalmic device; priming, by a priming mechanism of the device, the injection source and the extraction source simultaneously; activating the extraction source to extract a sample through a needle, the needle being in fluid communication with the extraction source of the device, and activating the injection source to inject an agent through the needle, the needle being in fluid communication with the injection source of the device.

The method of using the extraction and injection ophthalmic device does not form part of the invention.

Priming the injection source and the extraction source simultaneously may comprise translating a lever communicatively coupled to a spring, such that the spring is compressed to simultaneously prime both the injection source and the extraction.

The method may further comprise shearing, by shearing means, excess vitreous humor from the sample to be extracted.

Activating the extraction source may comprise depressing an extraction button of the device to extract the sample.

Activating the injection source may comprise depressing an injection button of the device to inject the agent.

The method may further comprise fluidically separating the extracted sample from the injection source.

### BRIEF DESCRIPTION OF THE DRAWINGS

Embodiments of the invention will be better understood and readily apparent to one of ordinary skill in the art from the following written description, by way of example only, and in conjunction with the drawings, in which:
Figs. 1A and 1B show perspective views of a device for ophthalmic extraction and injection, according to an example embodiment.
Fig. 1C shows a perspective view of the device of Fig. 1A with the cover opened, according to an example embodiment.
Fig. 1D shows a cross-sectional side view of the device of Fig. 1A, according to an example embodiment.
Fig. 2A shows a perspective view of the priming mechanism of the device of Fig. 1A, according to an example embodiment.
Fig. 2B shows a close up plan view of the priming mechanism of Fig. 2A.
Fig. 2C shows a plan view of the priming mechanism of Fig 2A.
Fig. 2D shows a perspective view of the priming mechanism of the device of Fig. 1A, according to an alternative embodiment.
Fig. 2E shows a close up plan view of the priming mechanism of Fig 2D.
Fig. 2F shows a plan view of the priming mechanism of Fig 2D.
Fig. 3 shows a cross-sectional functional view of the priming mechanism of the device of Fig. 1A, according to an example embodiment.
Figs. 4A-4B show perspective views of a button cover for the injection and extraction button of the device of Fig. 1A, according to an alternative embodiment.
Fig. 5 shows a flow chart illustrating a method for ophthalmic extraction and injection.

### DETAILED DESCRIPTION

The following detailed description is merely exemplary in nature and is not intended to limit the invention or the application and uses of the invention. Furthermore, there is no intention to be bound by any theory presented in the preceding background of the invention or the following detailed description. Herein, devices and methods for ophthalmic extraction and injection are presented in accordance with present embodiments having the advantages of providing a safe and reliable way to extract vitreous and inject anti-VEGF agents in a single injection. In addition, it may also reduce intraocular pressure spikes, improve accuracy of drug delivery and prevent reflux of vitreous material which may lower the risk of damage to ocular structures.

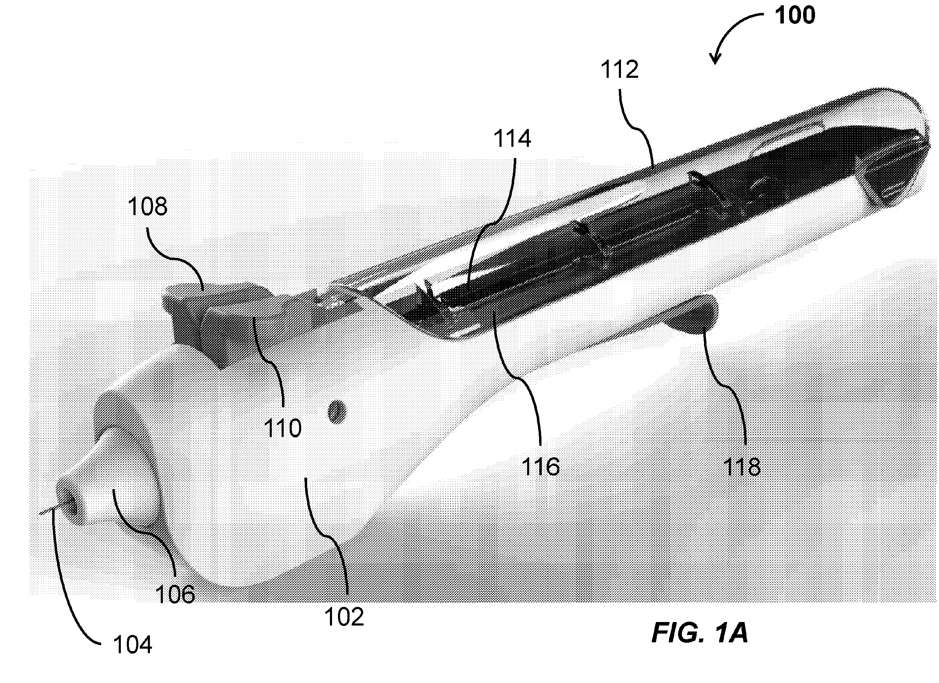

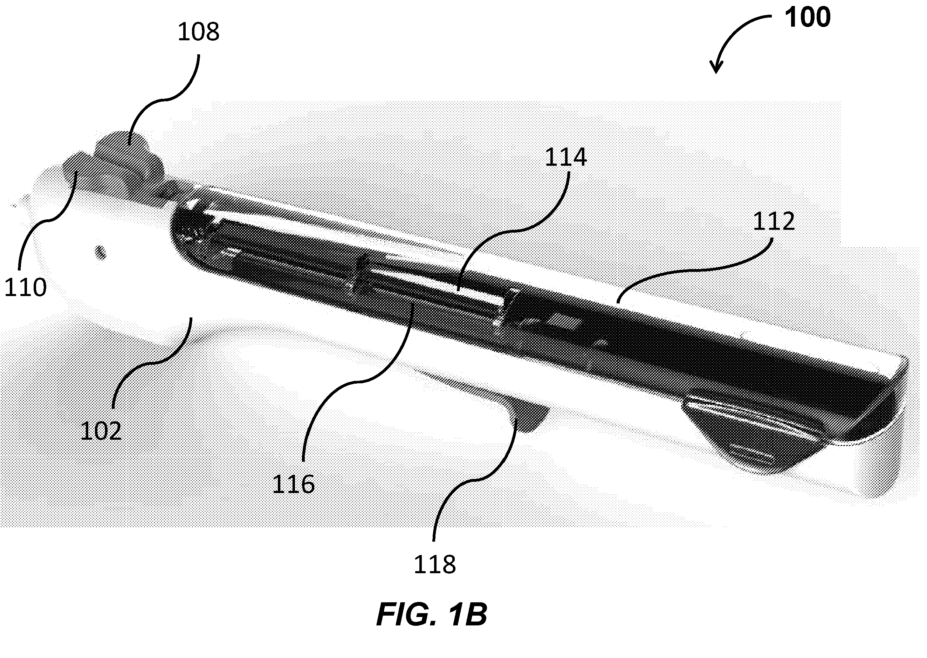

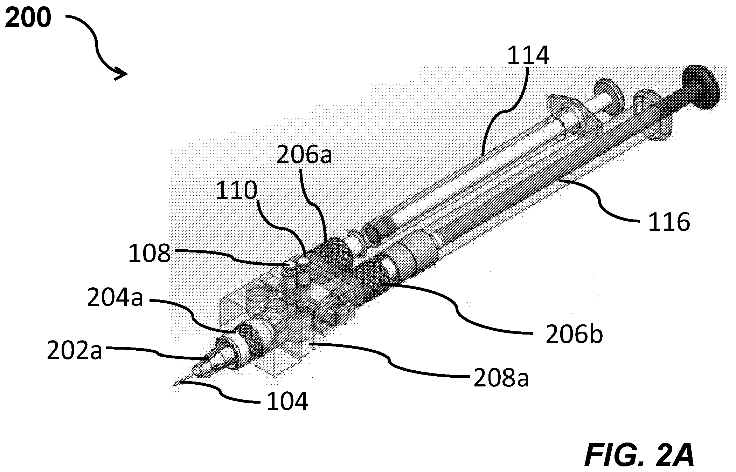

Figs. 1A and 1B show perspective views of a device 100 for ophthalmic extraction and injection, according to an example embodiment. The device 100 includes a housing 102, a needle 104, a nozzle 106, an injection button 108, an extraction button 110 and a cover 112. The housing 102 may include an injection portion, an extraction portion and a priming mechanism. A detailed view of the priming mechanism, the injection portion and the extraction portion are shown in Figs. 2A-2D and will be explained in more detail below. The injection portion may be configured to receive an injection source 114 while the extraction portion may be configured to receive an extraction source 116. In an embodiment, the injection portion may be adjacent to the extraction portion such that the injection source 114 and the extraction source 116 are positioned side-by-side. In an alternative embodiment, the injection portion may be above or below the extraction portion such that the injection source 114 and the extraction source 116 are positioned on top or below one another.

The needle 104 is selectively in fluid communication with the injection source 114 or the extraction source 116 when either the injection button 108 or the extraction button 110 is depressed. The injection source 114 may be a syringe which can contain an agent that is used to treat retinal disease. The injection portion, together with the injection source 114, is in fluid communication with the needle 104 such that on activation of the injection source 114 when the injection button 108 is depressed, the device 100 is configured to inject the agent from the injection source 114 through the needle 104.

The extraction source 116 may include any one of: a syringe, a vacutainer or a pump. The extraction portion, together with the extraction source 116, is in fluid communication with the needle 104 such that on activation of the extraction source 116 when the extraction button 110 is depressed, the device 100 is configured to extract a sample through the needle 104 to the extraction source 116. The extraction source 116 is also fluidically separated from the injection source 114 such that, in use, the agent in the injection source 114 does not contact the sample to be extracted. In this way, contamination of the extracted sample and the agent may be prevented which can advantageously result in providing a safe and reliable way to extract vitreous sample and inject anti-VEGF agents.

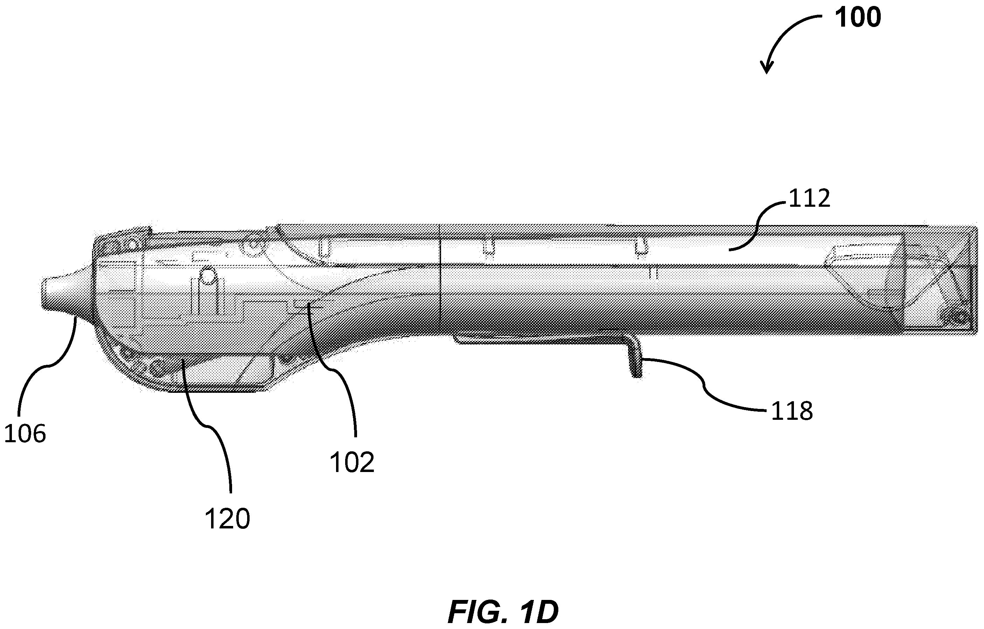

The priming mechanism includes a lever 118 and a spring 120 (as shown in Fig. 1D) such that the lever 118 is communicatively coupled to the spring and a translation movement of the lever 118 is configured to compress the spring to simultaneously prime both the injection source 114 and the extraction source 116. The priming mechanism may also include a plurality of valves which are in communication with the injection source 114 and the extraction source 116 whereby the plurality of valves are closed to prevent contamination of the agent when the injection source 114 and the extraction source 116 are primed. The plurality of valves may include at least one duckbill valve.

The cover 112 may be pivotably mounted to the housing 102 such that the injection source 114 and the extraction source 116 can be attached or removed from the housing 102 when the cover 112 is in an open position. As shown in the Figures, the cover 112 is at a closed position after the injection source 114 and the extraction source 116 are attached to the housing 102. The cover 112 may also include a tab which interacts with the injection button 108 and the extraction button 110 such that the plurality of valves in the priming mechanism are open when the cover 112 is at the open position.

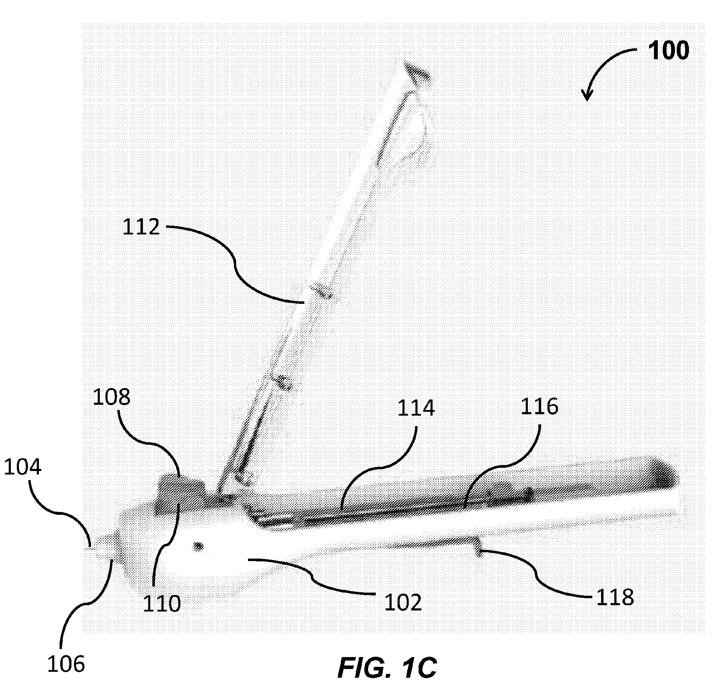

Fig. 1C shows a perspective view of the device of Fig. 1A with the cover 112 opened, according to an example embodiment. In the Figure, the cover 112 is in the open position with the injection source 114 and the extraction source 116 attached to the housing 102. At the open position, the injection source 114 and the extraction source 116 may also be removed from the housing 102. Fig. 1D shows a cross-sectional side view of the device of Fig. 1A, according to an example embodiment. The device 100 as shown in the Figure has the cover 112 in a closed position and the needle 104 retracted into the housing 102. The spring 120 of the priming mechanism is at an uncompressed position in the Figure as the injection source and the extraction source have yet to be attached to the housing 102.

The nozzle 106 is arranged to receive the needle 104 so that the needle 104 can selectively protrude from the housing 102 when the priming mechanism is ready for priming. It can be appreciated that the needle 104 may retract into the housing 102 via the nozzle 106 when the agent from the injection source 114 has been delivered and the sample has been extracted into the extraction source 116. The housing 102 may also include a cap (not shown) that is configured to cover the nozzle 106 and the needle 104 for safety reasons when the needle 104 is protruding from the housing 102 even though the device 100 is not in use.

The device 100 may include shearing means configured to shear excess vitreous humor from the sample to be extracted by the extraction source 116. The shearing means may be part of the needle 104 or may be positioned aft of the needle 104 to shear the excess vitreous humor after the sample is extracted from the needle 104.

Fig. 2A shows a perspective view of the priming mechanism 200 of the device of Fig. 1A, according to an example embodiment. As shown in the Figure, the priming mechanism 200 has a fitting 202a configured to receive the needle 104 and a collar 204a arranged to receive the fitting 202a. The priming mechanism 200 may also include a case 208a configured to receive the collar 204a, the injection button 108, the extraction button 110, the injection portion and the extraction portion.

In an embodiment, the priming mechanism 200 may include a first metal collar 206a that is configured to receive the injection portion and a second metal collar 206b configured to receive the extraction portion. The case 208a of the priming mechanism 200 is configured to receive the first and second metal collar 206a 206b. The fitting 202a of the priming mechanism 200 may be a metal luer fitting and the collar 204a may be a metal collar.

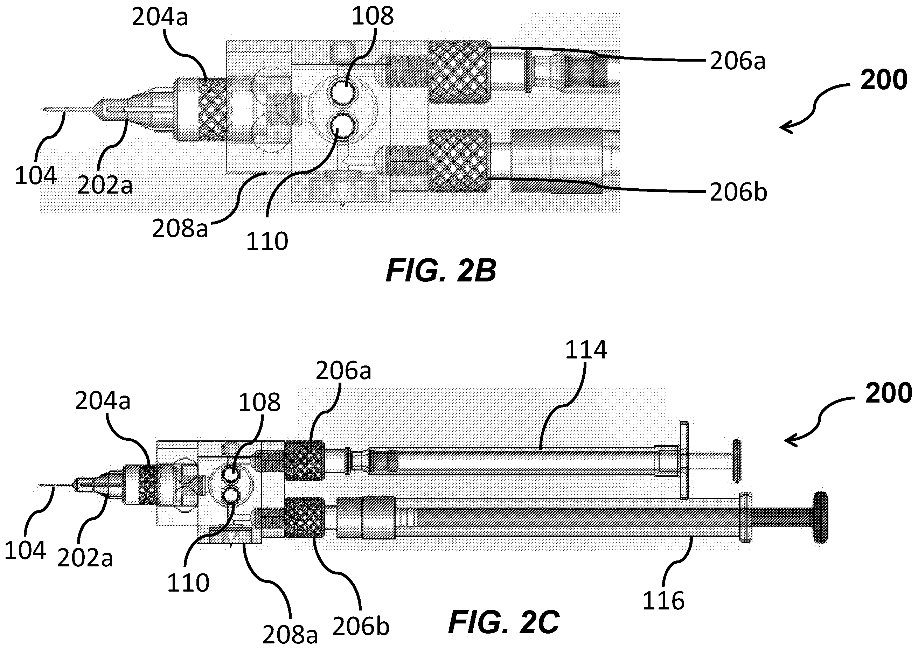

Fig. 2B shows a close up plan view of the priming mechanism of Fig. 2A, while Fig. 2C shows a plan view of the priming mechanism of Fig 2A. In the Figures, the case 208a of the priming mechanism 200 is arranged to receive the collar 204a, the first metal collar 206a, the second metal collar 206b, the injection button 108 and the extraction button 110.

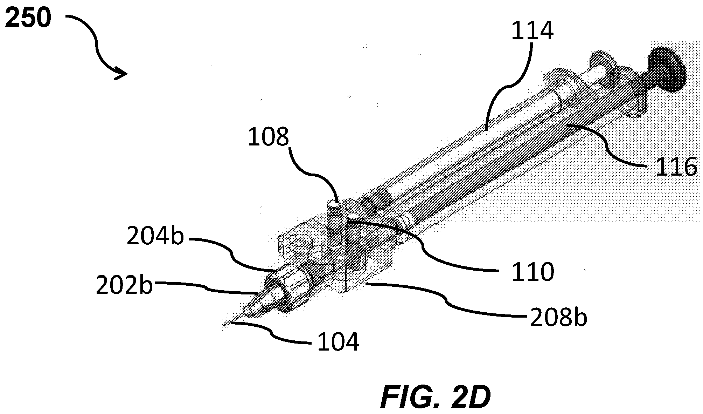

Fig. 2D shows a perspective view of the priming mechanism of the device of Fig. 1A, according to an alternative embodiment. As shown in the Figure, the priming mechanism 250 has a fitting 202b configured to receive the needle 104 and a collar 204b arranged to receive the fitting 202b. The priming mechanism 250 may also include a case 208b configured to receive the collar 204b, the injection button 108, the extraction button 110, the injection portion and the extraction portion.

In an embodiment, the fitting 202b of the priming mechanism 250 may be a plastic luer fitting while collar 204b may be a plastic collar. The priming mechanism 250 may also include slip fit leurs built into the injection portion and the extraction portion which can eliminate the need for the first and second metal collars 206a, 206b of the priming mechanism 200.

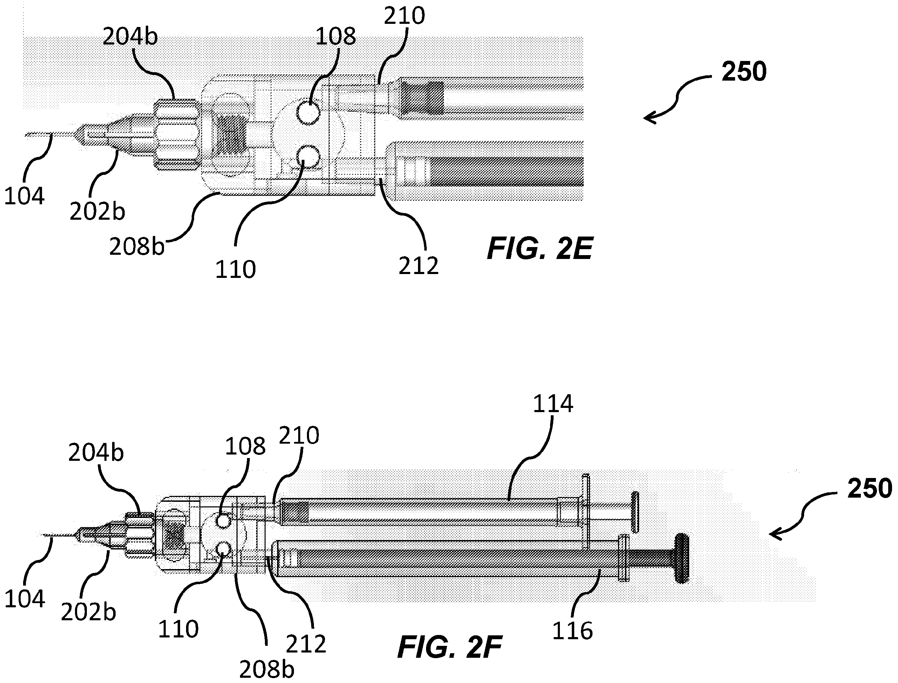

Fig. 2E shows a close up plan view of the priming mechanism of Fig 2D while Fig. 2F shows a plan view of the priming mechanism of Fig 2D. In the Figures, the case 208b of the priming mechanism 250 is arranged to receive the collar 204b, the injection portion 210, the extraction portion 212, the injection button 108 and the extraction button 110. The case 208a of the priming mechanism 200 (Figure 2A) is larger than the case 208b of the priming mechanism 250. The case 208b directly receives the injection portion 210 and the extraction portion 212 while the case 208a receives the injection portion and the extraction portions 210, 212 via the first metal collar 206a and the second metal collar 206b.

The distance between the injection button 108 and the extraction button 110 in priming mechanism 250 is more than the distance between the injection button 108 and the extraction button 110 in priming mechanism 200 (Figure 2A). The positioning of the plurality of valves (not shown) in priming mechanism 250 may also be different from those of priming mechanism 200. Each of the priming mechanisms 200, 250 may also include a plurality of internal ports configured to allow fluid communication between the needle 104 and the injection portion 210 or the extraction portion 212. By having a smaller case, having plastic fittings and slip fit luers, the priming mechanism 250 is smaller in size and weight as compared to priming mechanism 200. This may result in a reduction in the length, width and height of the device 100 which may advantageously lead to better ergonomics and better usability of the device 100 as the patient may feel more comfortable having a smaller device as compared to a larger device.

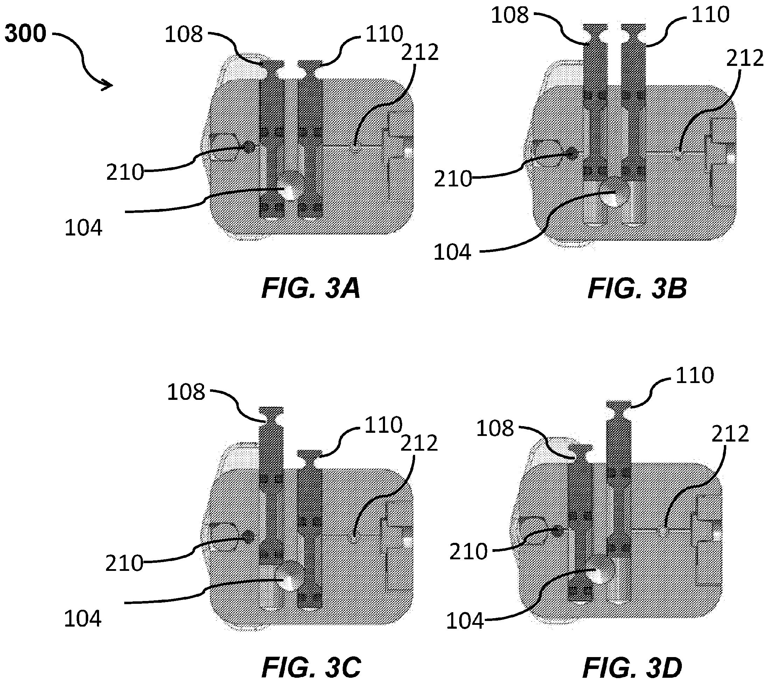

Figs. 3A-3D show cross-sectional functional views 300 of the priming mechanism of the device of Fig. 1A, according to an example embodiment. The priming mechanism may comprise a manifold of a double spool design having a low profile which may lead to a pen-styled grip design of the device 100. Having a pen-styled grip design may be advantageous in providing a better hold or grip of the device 100 in order to achieve a better control while operating the device 100 close to the patient. In Fig. 3A, the priming mechanism is at a state when the injection source 114 and the extraction source 116 are yet to be attached to the housing 102. The cover 112 of the housing 102 is at the open position and both the injection button 108 and the extraction button 110 are depressed. The plurality of valves (not shown) of the priming mechanism are open which may allow the needle 104 to be in communication with both the injection portion 210 and the extraction portion 212.

After the injection source 114 and the extraction source 116 are disposed in the housing 102 and the cover 112 is closed, the lever 118 is pulled away from the needle which compresses the spring 120 and simultaneously primes the injection source 114 and the extraction source 116. As shown in Fig 3B, the injection button 108 and the extraction button 110 are at an elevated position so that the needle 104 is not in communication with both the injection portion 210 and the extraction portion 212. At the same time, a vacuum is also created at the extraction portion 212.

In Fig 3C, the extraction button 110 is depressed to activate the extraction source 116, thereby only allowing communication between the needle 104 and the extraction portion 212. The vacuum previously created at the extraction portion 212 allows the extraction source 116 to extract the sample via the needle 104. Subsequently, after the sample has been extracted, the injection button 108 is depressed to activate the injection source 114. By depressing the injection button 108 after priming, the extraction button 110 returns to the elevated position as shown in Fig. 3D. This allows communication only between the needle 104 and the injection portion 210. The agent contained in the injection source 114 is thus delivered to the patient via the needle 104.

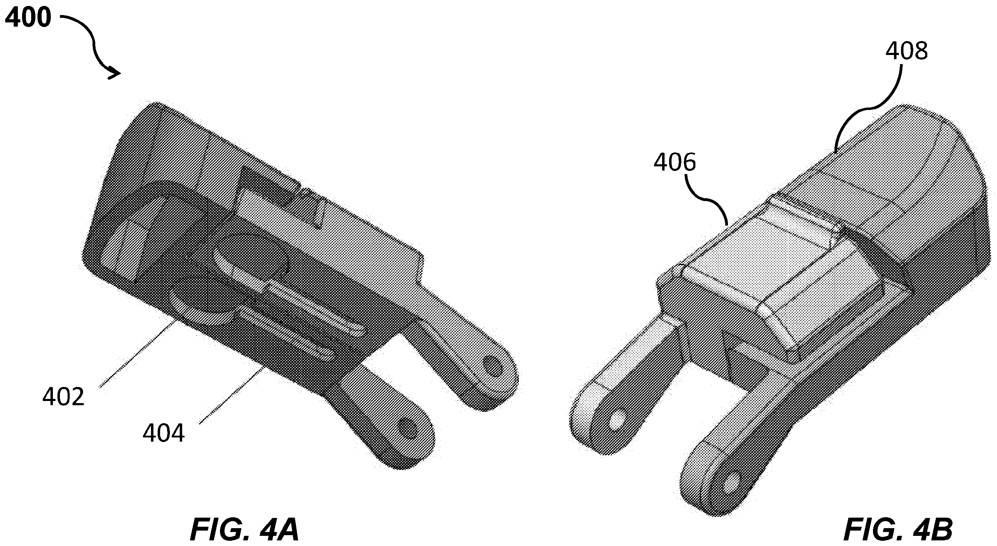

Figs. 4A-4B show perspective views of a button cover 400 for the injection and extraction button of the device of Fig. 1A, according to an alternative embodiment. The button cover 400 may include a spring well 402 configured to house the spring 120 of the priming mechanism and a spool slot 404. The button cover 400 in this embodiment may allow the injection button 406 and the extraction button 408 to adopt a front and back configuration, which can prevent the accidental depression of the injection button 406 or the extraction button 408. It can also prevent arching of a user's fingers over the injection and extraction buttons 406, 408 which may improve usability and ergonomics.

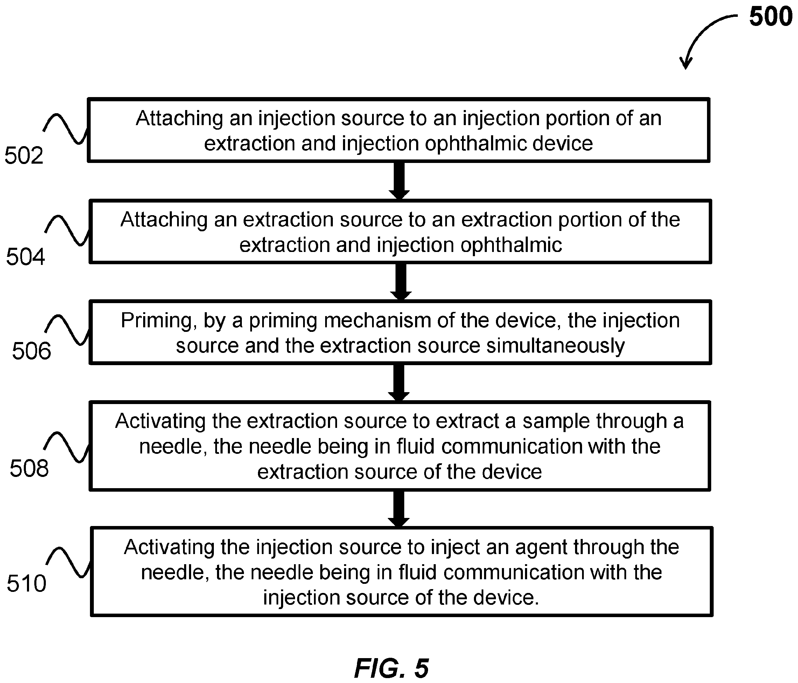

Fig. 5 shows a flow chart illustrating a method 500 for ophthalmic extraction and injection. The method comprises at step 502 attaching an injection source to an injection portion of an extraction and injection ophthalmic device. At step 504, the method includes attaching an extraction source to an extraction portion of the extraction and injection ophthalmic device. At step 506, the method includes priming, by a priming mechanism of the device, the injection source and the extraction source simultaneously. Priming the injection source and the extraction source simultaneously may include translating a lever communicatively coupled to a spring, such that the spring is compressed to simultaneously prime both the injection source and the extraction. At step 508, the method includes activating the extraction source to extract a sample through a needle, the needle being in fluid communication with the extraction source of the device. At step 510, the method includes activating the injection source to inject an agent through the needle, the needle being in fluid communication with the injection source of the device.

The method may further include shearing, by shearing means, excess vitreous humor from the sample to be extracted; activating the extraction source comprises depressing an extraction button of the device to extract the sample; and activating the injection source comprises depressing an injection button of the device to inject the agent.

The device and method for ophthalmic extraction and injection as described herein may be used to perform biopsy and injection simultaneously with only a single device. The injection and extraction sources may be disposable and compatible with standard therapeutics and syringe fittings. The device and method as disclosed may maintain intraocular pressure during injection of the agent and may have a low risk of retinal detachment during vitrectomy, i.e. extraction of the vitreous humor gel of a patient. The device and method may also result in little or no damage to the lens or retinal structures while having a clean extraction of vitreous humor. The device and method may also be used to monitor Vascular Endothelial Growth Factor levels from biopsy for patient-tailored therapy.

While exemplary embodiments have been presented in the foregoing detailed description of the invention, it should be appreciated that a vast number of variations exist.

It should further be appreciated that the exemplary embodiments are only examples, and are not intended to limit the scope, applicability, operation, or configuration of the invention in any way. Rather, the foregoing detailed description will provide those skilled in the art with a convenient road map for implementing an exemplary embodiment of the invention, it being understood that various changes may be made in the function and arrangement of elements and method of operation described in an exemplary embodiment without departing from the scope of the invention as set forth in the appended claims.

It will be appreciated by a person skilled in the art that numerous variations and/or modifications may be made to the present invention as shown in the specific embodiments without departing from the scope of the invention as defined by the appended claims. The present embodiments are, therefore, to be considered in all respects to be illustrative and not restrictive.

## Claims

1. An extraction and injection ophthalmic device (100) comprising:
a housing (102) comprising:
an injection portion configured to receive an injection source (114);
an extraction portion configured to receive an extraction source (116); and
a priming mechanism (200, 250) configured to prime both the injection source (114) and the extraction source (116); and
a needle (104) selectively in fluid communication with the injection source (114) or the extraction source (116);
wherein on activation of the extraction source (116), the device (100) is configured to extract a sample through the needle (104), and on activation of the injection source (114), the device (100) is configured to inject an agent through the needle (104),
**characterized in that** the priming mechanism (200, 250) comprises a lever (118) communicatively coupled to a spring (120), such that a translation movement of the lever (118) is configured to compress the spring (120) to simultaneously prime both the injection source (114) and the extraction source (116).

2. The device (100) according to claim 1, further comprising a manifold configured to selectively connect the needle (104) with the injection source (114) or the extraction source (116), wherein the manifold comprises a double spool manifold.

3. The device (100) according to claim 1 or 2, further comprising shearing means configured to shear excess vitreous humor from the sample to be extracted.

4. The device (100) according to any one of the preceding claims, wherein the injection source (114) and the extraction source (116) are removably attached to the housing (102).

5. The device (100) according to any one of the preceding claims, wherein the injection source (114) includes a syringe, and wherein the extraction source (116) includes any one of: a syringe, a vacutainer or a pump.

6. The device (100) according to any one of the preceding claims, further comprising an injection button (108) configured to activate the injection source (114) such that the injection portion is in fluid communication with the needle (104) to inject the agent from the injection source (114) to the needle (104).

7. The device (100) according to any one of the preceding claims, further comprising an extraction button (110) configured to activate the extraction source (116) such that the extraction portion is in fluid communication with the needle (104) to extract the sample from the needle (104) to the extraction source (116).

8. The device (100) according to any one of the preceding claims, wherein the extraction source (116) is fluidically separated from the injection source (114) such that, in use, the agent does not contact the sample.

## Patentansprüche

1. Eine ophthalmische Extraktions- und Injektionsvorrichtung (100), die folgende Merkmale aufweist:
ein Gehäuse (102), das folgende Merkmale aufweist:
einen Injektionsabschnitt, der dazu konfiguriert ist, eine Injektionsquelle (114) aufzunehmen;
einen Extraktionsabschnitt, der dazu konfiguriert ist, eine Extraktionsquelle (116) aufzunehmen; und
einen Füllmechanismus (200, 250), der dazu konfiguriert ist, sowohl die Injektionsquelle (114) als auch die Extraktionsquelle (116) zu füllen; und
eine Nadel (104), die selektiv in Fluidkommunikation mit der Injektionsquelle (114) oder der Extraktionsquelle (116) steht;
wobei bei Aktivierung der Extraktionsquelle (116) die Vorrichtung (100) dazu konfiguriert ist, eine Probe durch die Nadel (104) zu extrahieren, und bei Aktivierung der Injektionsquelle (114) die Vorrichtung (100) dazu konfiguriert ist, ein Mittel durch die Nadel (104) zu injizieren,
**dadurch gekennzeichnet, dass** der Füllmechanismus (200, 250) einen Hebel (118) aufweist, der kommunikativ mit einer Feder (120) gekoppelt ist, so dass eine Translationsbewegung des Hebels (118) dazu konfiguriert ist, die Feder (120) zusammenzudrücken, um gleichzeitig sowohl die Injektionsquelle (114) als auch die Extraktionsquelle (116) zu füllen.

2. Die Vorrichtung (100) gemäß Anspruch 1, die ferner einen Verteiler aufweist, der dazu konfiguriert ist, die Nadel (104) selektiv mit der Injektionsquelle (114) oder der Extraktionsquelle (116) zu verbinden, wobei der Verteiler einen Doppelspulenverteiler aufweist.

3. Die Vorrichtung (100) gemäß Anspruch 1 oder 2, die ferner eine Schereinrichtung aufweist, die dazu konfiguriert ist, überschüssige Glaskörperflüssigkeit von der zu extrahierenden Probe abzuscheren.

4. Die Vorrichtung (100) gemäß einem der vorhergehenden Ansprüche, wobei die Injektionsquelle (114) und die Extraktionsquelle (116) entfernbar an dem Gehäuse (102) angebracht sind.

5. Die Vorrichtung (100) gemäß einem der vorhergehenden Ansprüche, wobei die Injektionsquelle (114) eine Spritze umfasst, und wobei die Extraktionsquelle (116) eines der Folgenden umfasst: eine Spritze, einen Vacutainer oder eine Pumpe.

6. Die Vorrichtung (100) gemäß einem der vorhergehenden Ansprüche, die ferner einen Injektionsknopf (108) aufweist, der dazu konfiguriert ist, die Injektionsquelle (114) so zu aktivieren, dass der Injektionsabschnitt in Fluidkommunikation mit der Nadel (104) steht, um das Mittel von der Injektionsquelle (114) zu der Nadel (104) zu injizieren.

7. Die Vorrichtung (100) gemäß einem der vorhergehenden Ansprüche, die ferner einen Extraktionsknopf (110) aufweist, der dazu konfiguriert ist, die Extraktionsquelle (116) so zu aktivieren, dass der Extraktionsabschnitt in Fluidkommunikation mit der Nadel (104) steht, um die Probe von der Nadel (104) zu der Extraktionsquelle (116) zu extrahieren.

8. Die Vorrichtung (100) gemäß einem der vorhergehenden Ansprüche, wobei die Extraktionsquelle (116) fluidisch von der Injektionsquelle (114) getrennt ist, so dass das Mittel bei der Verwendung die Probe nicht kontaktiert.

## Revendications

1. Dispositif (100) ophtalmique d'extraction et d'injection comprenant :
un boîtier (102) comprenant :
une partie d'injection configurée pour recevoir une source d'injection (114) ;
une partie d'extraction configurée pour recevoir une source d'extraction (116) ; et
un mécanisme d'amorçage (200, 250) configuré pour amorcer à la fois la source d'injection (114) et la source d'extraction (116) ; et
une aiguille (104) sélectivement en communication fluidique avec la source d'injection (114) ou la source d'extraction (116) ;
dans lequel, à l'activation de la source d'extraction (116), le dispositif (100) est configuré pour extraire un échantillon à travers l'aiguille (104), et à l'activation de la source d'injection (114), le dispositif (100) est configuré pour injecter un agent à travers l'aiguille (104),
**caractérisé en ce que** le mécanisme d'amorçage (200, 250) comprend un levier (118) couplé en communication à un ressort (120), de telle sorte qu'un mouvement de translation du levier (118) est configuré pour comprimer le ressort (120) pour amorcer simultanément à la fois la source d'injection (114) et la source d'extraction (116).

2. Dispositif (100) selon la revendication 1, comprenant en outre un collecteur configuré pour relier sélectivement l'aiguille (104) à la source d'injection (114) ou à la sourcer d'extraction (116), dans lequel le collecteur comprend un collecteur à double tiroir.

3. Dispositif (100) selon la revendication 1 ou 2, comprenant en outre un moyen de cisaillement configuré pour cisailler l'excès d'humeur vitrée de l'échantillon à extraire.

4. Dispositif (100) selon l'une quelconque des revendications précédentes, dans lequel la source d'injection (114) et la source d'extraction (116) sont attachées de manière amovible au boîtier (102).

5. Dispositif (100) selon l'une quelconque des revendications précédentes, dans lequel la source d'injection (114) inclut une seringue, et dans lequel la source d'extraction (116) inclut l'un quelconque parmi : une seringue, un tube Vacutainer ou une pompe.

6. Dispositif (100) selon l'une quelconque des revendications précédentes, comprenant en outre un bouton d'injection (108) configuré pour activer la source d'injection (114) de telle sorte que la partie d'injection est en communication fluidique avec l'aiguille (104) pour injecter l'agent depuis la source d'injection (114) vers l'aiguille (104).

7. Dispositif (100) selon l'une quelconque des revendications précédentes, comprenant en outre un bouton d'extraction (110) configuré pour activer la source d'extraction (116) de telle sorte que la partie d'extraction est en communication fluidique avec l'aiguille (104) pour extraire l'échantillon de l'aiguille (104) vers la source d'extraction (116).

8. Dispositif (100) selon l'une quelconque des revendications précédentes, dans lequel la source d'extraction (116) est fluidiquement séparée de la source d'injection (114) de telle sorte que, lors de l'utilisation, l'agent n'entre pas en contact avec l'échantillon.
